# EUROPEAN PATENT APPLICATION

(11) **EP 2 511 011 A2**
(43) Date of publication of application: **17.10.2012**
(21) Application number: 10836248.4
(22) Date of filing: 15.12.2010
(51) Int. Cl.: B04B 1/10, C12M 1/10

(54) **CENTRIFUGE TUBE**

(30) Priority: 07.12.2009 KR 20090120282; 19.12.2009 KR 20090127565; 18.01.2010 KR 20100004358
(71) Applicant: Jeon, Min-Yong, Seoul 135-998 (KR)
(72) Inventor: Jeon, Min-Yong, Seoul 135-998 (KR)
(74) Representative: Garavelli, Paolo
(86) International application number: PCT/KR2010/008968
(87) International publication number: WO 2011/071353

(57) **Abstract**

The present invention relates to a centrifuge tube for use in the centrifugation of biological materials, wherein the diameter of the centrifuge tube is improved to enable minute centrifugation, and in which a thin, weak, and narrow portion of the centrifuge tube, to which a strong force is to be applied, is provided with a support piece to enable centrifugation to be performed without causing a deformation in the centrifuge tube. The centrifuge tube of the present invention enables the centrifugation of materials using differences in the specific gravities of the materials to be easily performed without using a reagent or the like. The centrifuge tube of the present invention has a closure made of a material such as a rubber, polyethylene, polypropylene, silicon or the like, such that a needle may penetrate through the closure and the centrifuge tube can be protected from contamination caused by bacteria, and centrifugation of biological materials can be performed in a closed space.

## Description

### TECHNICAL FIELD

The present invention relates to a centrifuge tube, more particularly, a centrifuge tube allowing easy separation of a specific component.

### BACKGROUND ART

For all of biology, genetics or medical science etc., separation of biological substance may be said to be most fundamental operation for not only analysis of substance but also cultivation of cells, and identification and amplification of DNA. Currently, in the field of medical science, researches are competitively conducted in regenerative medicines using stem cells. For obtaining such stem cells, there are embryonic stem cell obtained from blistocyst of initial stage of generation and adult stem cells obtained from adult or placeta whose generation process has been terminated.

There are three main methods of obtaining the adult stem cells among the cells; first, a method of obtaining the adult stem cells through bone marrow, second, a method of obtaining it through umbilical cord blood, and third, a method of obtaining it through peripheral blood. In all these methods, components (erythrocyte, leukocyte, blood platelet and blood plasma) of the blood are first separated by means of centrifugation, followed by an operation of separating the stem cell (mononuclear cell). In this connection, conventional centrifugation methods will be contemplated as follows.

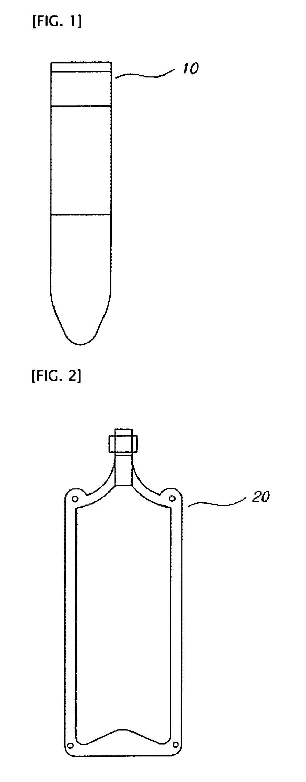

Fig. 1 is a view showing an example of conventional centrifuge tube, wherein if solution containing various mixed substances, particularly, the blood of human being or animal is extracted to be centrifuged, erythrocyte, leukocyte, blood platelet and other blood plasma in the blood are positioned in layers from bottom to top in weight order due to their inherent difference in specific gravity.

First, for a process of passing the peripheral blood extracted through syringe needle to the centrifuge tube, a stopper of the centrifuge tube is opened, the needle of the syringe is positioned inside the centrifuge tube, and then the peripheral blood in the syringe is passed to the centrifuge tube, and thereafter the needle is removed from the centrifuge tube and then the opened centrifuge tube is closed with the stopper, and the centrifuge tube is subsequently positioned in bucket of the centrifugal separator, and the separator is operated by setting desired time, rotational speed(rpm) or acceleration of gravity(g). Usually, the erythrocyte has the largest specific gravity among the components of the blood and thus are collected in the bottom portion of the centrifuge tube, and the erythrocyte account for 47% of the whole blood for man and 42% for woman. Positioned above the erythrocyte are the leukocyte and blood platelet, which account for about 1% of the whole blood, and in which monocytes (stem cells of the peripheral blood) are also included. Serum and fibrinogen referred to as blood plasma as remainder are positioned above the leukocyte and blood platelet. The centrifuge tube for which centrifugation has been terminated is removed and the stopper is then opened again, and desired substance among the substances separated due to difference in specific gravity of the component substances is separated by means of a pipette or capillary tube or needle, and thereafter the remaining components are separately stored or discarded or re-cycled.

In such an operation process, during a step of opening and closing the stopper and a step of charging the blood, a concern occurs that foreign material such as virus or other bacteria etc. in the air penetrates into centrifuge tube, and if the foreign material is cultivated, contaminated culture is naturally produced. Furthermore, the centrifuge tube used at this time is formed in such a manner that its diameter generally becomes narrower downward. In such a case, if desired component is present at the bottom portion, its separation can be easily carried out, however, if desired component is not present at the bottom portion in the whole solution, but present in an intermediate position or higher position in a section of larger diameter of the centrifuge tube according to the specific gravity, inconvenience occurs that the desired component cannot be finely separated. In particular, buffy coat layer containing the stem cells to be separated account for only about 1% of the whole solution, therefore, when the buffy coat layer is to be separated in the centrifuge tube of substantially large diameter, since the buffy coat layer is thinly laid over the erythrocyte like a paper, it is very difficult for even experts to separate the layer.

Fig. 2 is a view showing an example of blood extracting bag usually referred to as a blood bag. The blood collected through blood-gathering is temporarily stored in the bag and centrifuged so that respective component, i.e., the erythrocyte, leukocyte, blood platelet and blood plasma is separated. For the above-mentioned two containers, when the leukocyte or blood platelet is to be separated, if these components accounting for about 1% of the whole blood is first centrifuged, they form thin buffy coat layer whose surface is white, and separation of such a buffy coat layer causes a very complicated problem in actual clinical stage.

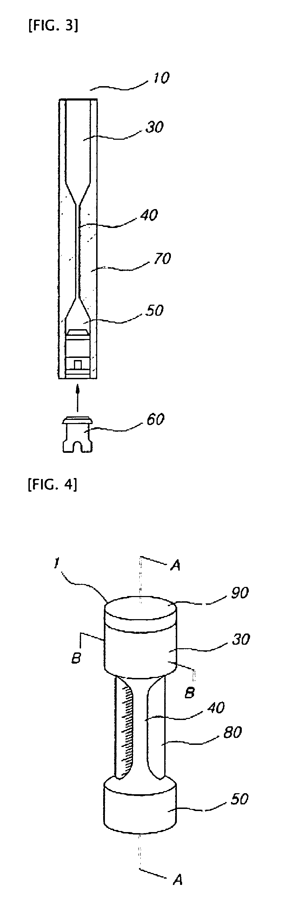

Fig. 3 is a sectional view of yet another conventional centrifuge tube, wherein for the diameter of the centrifuge tube (10), the diameter of intermediate chamber (40) is remarkably small compared to other two chambers (30, 50). The buffy coat layer is very easy to separate when the layer is formed in such a slim intermediate chamber (40) compared to when the layer is formed in other two chambers (30, 50). However, since the ratio of the erythrocyte in the whole blood is different between man and woman or among individuals as described above, a separate plug (60) is provided as means for moving the buffy coat layer to the intermediate chamber (40), and a separate block (70) surrounding the centrifuge tube (10) is provided in order to prevent the intermediate chamber (40) with a small diameter from being deformed due to high acceleration of gravity (g) generated at the time of centrifugation.

In particular, according to US Pat. No. 3,513,976, intermediate portion of the centrifuge tube is constricted like a neck in order to obtain the leukocyte in the peripheral blood, and the erythrocyte is collected in lower chamber, a hole and another tube are provided on one surface of the lower chamber such that the leukocyte can be positioned in the neck portion. This method requires high production cost and is troublesome to use.

In addition, according to US Pat. No. 4,861,477, in order to eliminate these drawbacks, a plug is provided on bottom side of the centrifuge tube to adjust the volume of a space in which the erythrocyte is separated, and in order to prevent a constricted intermediate portion of the centrifuge tube from being deformed due to high acceleration of gravity, another block structure is provided on outside the centrifuge tube, which increases production cost of the centrifuge tube.

Furthermore, according to US Pat. No. 5,422,018, medium is filled between inner centrifuge tube whose intermediate portion is concave and outer centrifuge tube surrounding the inner centrifuge tube, thereby serving to protect the intermediate chamber portion of the inner centrifuge tube which has a small diameter, however, this method is also troublesome to operate and increases production cost of the centrifuge tube.

Korean Patent Application Publication No. 10-2006-0059849 discloses a method for producing platelet rich plasma comprising a process of adding water-soluble polymer to the peripheral blood, and for similar methods, ficoll-hypaque, i.e., copolymer of ficoll (sucrose polymer) and hypaque (polymer of sodium ditrizote) is utilized in order to separate monocytes from peripheral blood in laboratory. This ficoll-hypaque has specific gravity of 1.0778 g/ml, and since monocyte is lighter than the former, while the erythrocyte is heavier than it, separation is carried out using the difference in the specific gravity. In the case of utilizing the above-mentioned polymer, it is added to aMEM (alpha Minimum Essential Medium available from Jaeil Biotech Service in Korea) for washing, and then centrifugation is carried out for 10 minutes and at 200xg, followed by a washing process in which aMEM is discarded except for cells settled on the bottom of falcon tube.

Since solid components contained in the peripheral blood or other biological substances and all kinds of liquid are different in specific gravity, centrifuging method utilizing such a difference in its specific gravity is most economical.

By the way, although there are products wherein the portion of the centrifuge tube in which a component of minute amount is to be positioned using minute difference in specific gravity has small diameter, they are hard to produce and have a risk of contamination, and are troublesome to use, and as additional device or expensive material is used, there is a problem of high production cost, and they are not suitable for use in the bucket of the centrifugal separator to which currently often conical tubes of 15 ml and 50 ml-type are applied.

### [Patent Documents]

[Document 1] US 3,513,976 (W.C. James) 1970.5.26
[Document 2] US 4,861,477 (Shiro Kimura) 1989.8.29
[Document 3] US 5,422,018 (Alexander M. Saunders and other two) 1995.6.6
[Document 4] KR 10-0,409,028 (Jeon Hong Jae) 2003.11.27
[Document 5] KR 10-0,489,248 (MEDIPOST Corp.) 2005.5.11
[Document 6] KR 10-2006-0,059,849 (Smida Emi) 2006.6.2
[Document 7] US 6,835,353 (Emery Smith and other two) 2004.12.28

### DISCLOSURE OF THE INVENTION

The present invention aims at providing a centrifuge tube allowing easy separation of a specific component from blood sample to be centrifuged.

The present invention further aims at providing a centrifuge tube allowing efficient separation of components from solution containing biological or chemical components, including peripheral blood, by means of difference in specific gravity of each substance contained in the solution.

In particular, the present invention further aims at providing a device for economical, safe and efficient separation of biological substance shielded from contamination, including biological contamination, occurring during a separating process in separation, cultivation and regeneration of cells researched in the recent medical science and biotechnology.

The present invention relates to a centrifuge tube having an outer wall for receiving flowable substance in inner space and for separating components of the flowable substance received in the inner space according to specific gravity, wherein the centrifugal tube comprises an upper chamber having an open top inlet and constant inner volume; an intermediate chamber communicating with the upper chamber at lower end thereof and having a smaller diameter than the upper chamber; and an lower chamber communicating with the intermediate chamber at lower end thereof and having a larger diameter than the intermediate chamber, and at least one support pieces are formed integrally with the an outer wall of the intermediate chamber in order to prevent the intermediate chamber from being deformed by centrifugal force at the time of centrifugation.

The present invention also relates to a centrifuge tube having an outer wall for receiving flowable substance in an inner space and for separating components of the flowable substance received in the inner space according to specific gravity, wherein the centrifugal tube comprises an upper chamber having an open top inlet and constant inner volume; and an intermediate chamber communicating with the upper chamber at lower end thereof and having a smaller diameter than the upper chamber, and in centrifugal separator in which 15 ml and 50 ml-type conical tubes are inserted, the volumes of the lower chamber, intermediate chamber and upper chamber are set in a predetermined ratio such that buffy coat layer can be collected in the intermediate chamber with a one-time centrifugation, and at least one support pieces are integrally formed on an outer wall of the intermediate chamber in order to prevent the intermediate chamber from being deformed at the time of centrifugation. Lower portions of the support pieces extend to a bucket of the centrifugal separator.

The present invention is characterized in that a stopper is provided on the upper chamber for closing open inlet of the upper chamber, which stopper is made of flexible material, particularly, one of rubber, silicone, PVC, polyethylene, polypropylene or polymer.

The present invention is characterized in that the stopper closing upper top end of the upper chamber is outwardly convex at its central portion, whereby disinfection can be easily carried out prior to insertion of a syringe needle. Furthermore, a settlement shoulder is formed on upper end of the upper chamber, and a cap is further provided which has a settlement groove engaging the settlement shoulder, thereby preventing easy separation of the stopper from the upper chamber.

The present invention is characterized in that the thickness of bottom surface of the lower chamber is smaller than that of side surface thereof, whereby the volume of the lower chamber can reduced if necessary in order to position minute amount of substance in the intermediate chamber.

The present invention also relates to a centrifugal pack having an outer shell for receiving flowable substance in inner space and for separating components of the flowable substance received in the inner space according to specific gravity, wherein the centrifugal pack comprises an upper chamber having an open top inlet and constant inner volume; an intermediate chamber communicating with the upper chamber at lower end thereof and having a smaller diameter than the upper chamber; and an lower chamber communicating with the intermediate chamber at lower end thereof and having a larger diameter than the intermediate chamber, and wherein hanging holes are formed in outer shell which forms the upper chamber, intermediate chamber and lower chamber, and these hanging holes are hung on a flat bucket having rings. Furthermore, it is also characteristic that a lower inlet is formed at a lower portion of the centrifugal pack.

The present invention is characterized in that in a centrifuge tube having an outer wall for receiving flowable substance in an inner space and for separating components of the flowable substance received in the inner space according to specific gravity, an outer wall of the intermediate chamber of the centrifuge tube is thicker than outer walls of the upper and lower chambers.

The present invention is characterized in that previously added into the centrifuge tube is a predetermined amount of fibrin activation agent such as thrombin, Calcium divalent ion, glass beads, or anticoagulant such as heparin, ACDA, CPDA, EDTA etc. or water-soluble polymeric compounds precipitating a specific substance, or separation material such as ficoll-hypaque etc., or targeted fluorescent material-separating reagent utilizing antigen-antibody reaction or magnetic microbeads.

The present invention is characterized in that indication part for indicating the volume of the object to be centrifuged is formed on outer surface of the upper chamber.

The present invention also relates to a centrifuge tube having an upper chamber, intermediate chamber and lower chamber, wherein the volume of the lower chamber accounts for about 36% of the total volume of the centrifuge tube, and the volume of the intermediate chamber accounts for about 10 ± 2% of the total volume, thus the component to be extracted is collected in the intermediate chamber.

The present invention has the following effects.

Upon centrifugation by means of difference in specific gravity of biological or chemical substance to be separated, due to the support pieces formed on side surface of the centrifuge tube, the centrifuge tube with a small diameter has a capability to endure high centrifugal force and acceleration of gravity generated at the time of centrifugation, whereby as fine as possible centrifugation is enabled by means of difference in specific gravity only using centrifuge tube with a small diameter. For example, by using the centrifuge tube according to difference in specific gravity and compositional ratio of the peripheral blood to be separated, the buffy coat layer including targeted stem cells is formed in the intermediate chamber with a small diameter, whereby separation of the buffy coat layer can be efficiently carried out.

Due to the simply formed support pieces, effects are obtained that inconvenience is eliminated of forming a separate outer block or installing intermediate medium, unlike existing methods, thus the centrifuge tube can be produced with a low cost, and fine centrifugation is enabled even without a troublesome washing process for removing artificial polymer after use thereof, and hence easy operation in laboratory is allowed.

Since the bottom surface of the lower chamber of the centrifuge tube is slightly thin to be so flexible that solution formed in the lower chamber can be pushed into the intermediate chamber even without providing a separate screw or plug, the substance to be separated can be positioned in the intermediate chamber with a small diameter.

If the centrifugal container is in the form of a pack, substances of the blood can be easily separated without moving the blood into a separate container, and when desired substance is present in the lower chamber, if the lower chamber is simply pushed, the desired substance is moved into the intermediate chamber and thereafter can be separated, and in the primarily centrifuged blood pack, the desired substance can be separated from a layer including erythrocyte and other substances in the intermediate chamber by means of forceps or welding machine, whereby it is not necessary to separately store them in a separate container, and they can be easily separated by providing a blood-withdrawing stopper on the side of erythrocyte, if necessary.

When biological or chemical substance is charged into the centrifuge tube or centrifuged substance is separated and then extracted, by providing a stopper penetrated by a needle, an advantage is obtained that centrifugal operation can be carried out while being protected from biological contamination occurring upon opening of the stopper.

When the centrifuge tube with a small diameter is removed from the centrifugal separator, due to the support pieces, the centrifuge tube is prevented from falling down, whereby separating operation of the centrifuged substance can be carried out even while the tube is erected on a floor.

Since indication lines or indication characters or indication symbols etc. are marked on a surface of the centrifuge tube for differently indicating the volume of charged blood for man or woman, even if operator simply charges the blood only up to the indication line, the buffy coat layer including the desired stem cells can be separated in the intermediate chamber.

In the centrifugal separator employing 15 ml-type conical tube which is currently most widely used in various medical institutions or laboratory etc., the desired substance, i.e., buffy coat can be easily extracted from the blood with a one-time centrifugation.

For the centrifuge tube of the present invention, the volume of the lower chamber accounts for about 36% of the total volume of the centrifuge tube, and the volume of the intermediate chamber accounts for about 10 ± 2% of the total volume, whereby if the blood is centrifuged, the buffy coat layer to be extracted is collected in the intermediate chamber, thus is very easy to extract.

### BRIEF DESCRIPTION OF THE DRAWINGS

Fig. 1 is a view showing an example of conventional centrifuge tube.
Fig. 2 is a view showing an example of conventional blood extracting bag.
Fig. 3 is a sectional view showing an example of conventional centrifuge tube.
Fig. 4 is a perspective view showing an example of centrifuge tube of the present invention.
Fig. 5 is a perspective view showing an example of centrifuge tube of the present invention.
Fig. 6 is a sectional view taken along line A - A in Fig. 4.
Fig. 7 is a sectional view taken along line C - C in Fig. 5.
Fig. 8 is a sectional view taken along line B - B in Fig. 4.
Fig. 9 is a sectional view taken along line D - D in Fig. 5.
Fig. 10 is a sectional view showing an example of the centrifuge tube of the present invention.
Fig. 11 is a sectional view showing an example of the centrifuge tube of the present invention.
Fig. 12 is a view explaining an example of blood extracting bag of the present invention.
Fig. 13 is a view explaining an example of blood extracting bag of the present invention.
Fig. 14 is a view explaining an example of blood extracting bag of the present invention.
Fig. 15 is a view explaining an example of blood extracting bag of the present invention.
Fig. 16 is a perspective view of a flat bucket of the present invention.
Fig. 17 is a perspective view of the centrifuge tube of the present invention.
Fig. 18 is a perspective view showing an example of the centrifuge tube of the present invention.
Fig. 19 is a sectional view taken along line A - A in Fig. 17.
Fig. 20 is a sectional view taken along line C - C in Fig. 18.
Fig. 21 is a sectional view taken along line B - B in Fig. 17.
Fig. 22 is a sectional view taken along line D - D in Fig. 17.
Fig. 23 is a perspective view showing an example of the centrifuge tube of the present invention.
Fig. 24 is an exploded perspective view showing an example of the centrifuge tube of the present invention.
Fig. 25 is a front view showing an example of the centrifuge tube of the present invention.
Fig. 26 is a sectional view taken along line A - A' in Fig. 25.

### MODES FOR CARRYING OUT THE INVENTION

With reference to Figs. 4 to 16, examples of the present invention will be described.

Fig. 4 shows an example of centrifuge tube of the present invention, wherein the centrifuge tube has a lower chamber (50) in which substance with the largest specific gravity is collected upon centrifugation, an intermediate chamber (40) in which substance with intermediate specific gravity is collected, an upper chamber in which substance with the smallest specific gravity is collected, and support pieces (80) which support the intermediate chamber next the same and connect with the upper and lower chambers. The support pieces (80) may be marked with scale for checking the volume of solution. There is formed at least one, preferably, three support pieces (80), and such support pieces (80) may be formed not only on side surface of the intermediate chamber (40) of the centrifuge tube, but also on side surface of the upper chamber (30) or lower chamber (50). Furthermore, the support pieces (80) may be marked with scale for checking the volume of solution. Furthermore, a stopper (90) may be provided on the top of the centrifuge tube, which stopper is made of chemical polymeric material such as rubber, silicone, polyethylene, polypropylene or PVC etc. and may be pierced by a needle. The stopper (90) may be coupled to the centrifuge tube (10) in a fitting manner or by forming threads on the centrifuge tube and also forming threads symmetrical to the threads of the tube on the stopper and screwing the stopper into the tube.

One of specific examples of the centrifuge tube of the present invention has numerical values as follows. By setting diameters and lengths of the upper chamber (30), intermediate chamber (40) and lower chamber (50) of the centrifuge tube in predetermined ratios, even when the centrifuge tube of the present invention is applied to all centrifugal separator in which conventional 15 ml and 50 ml-type centrifuge tubes are inserted, a desired component in the blood, i.e., buffy coat can be easily collected in the intermediate chamber (40) and easily extracted, without separate devices etc. That is, for outer diameter of the centrifuge tube, in the case of the centrifuge tube applied to centrifugal adaptor for 15 ml type, the outer diameter of the upper chamber (30) is kept at 16 mm ± 2 mm, and the outer diameter of the lower chamber (50) is also kept at 16 mm ± 2 mm, and the outer diameter of the intermediate chamber (40) is kept at 6 mm ± 2 mm, and the volume of the lower chamber (50) does not exceed 36 % of the total volume of the centrifuge tube, while the volume of the intermediate chamber (40) is 10% ± 2% of the total volume of the centrifuge tube. Furthermore, in the case of the centrifuge tube applied to centrifugal bucket for 50 ml type, the outer diameter of the upper chamber (30) is kept at 28 mm ± 2 mm, the outer diameter of the lower chamber (50) is also kept at 28 mm ± 2 mm, and the outer diameter of the intermediate chamber (40) is kept at 6 mm ± 2 mm, and the volume of the lower chamber (50) does not exceed 36 % of the total volume of the centrifuge tube, while the volume of the intermediate chamber (40) is 10% ± 2% of the total volume of the centrifuge tube. Therefore, the buffy coat is always collected in the intermediate chamber (40) at the time of centrifugation of the blood regardless of whether the support pieces (80) are present on the centrifuge tube or not.

In the case of centrifugation of the extracted blood etc. by using the centrifuge tube, erythrocyte layer with large specific gravity is collected in the lower chamber (50), buffy coat layer of leukocyte and blood platelet etc. is collected in the intermediate chamber (40), and the support pieces (80) prevent the intermediate chamber (40) from being deformed even when the high acceleration of gravity (g) is generated at the time of centrifugation. Furthermore, since biological liquid such as blood etc. can be charged into the centrifuge tube even without opening of the stopper (90), inclusion of virus or germ can be prevented. Therefore, closed operation can be performed in medical equipment or biological instrument requiring strict sterilization, whereby contamination of the specimen, culture or centrifuged substance can be prevented.

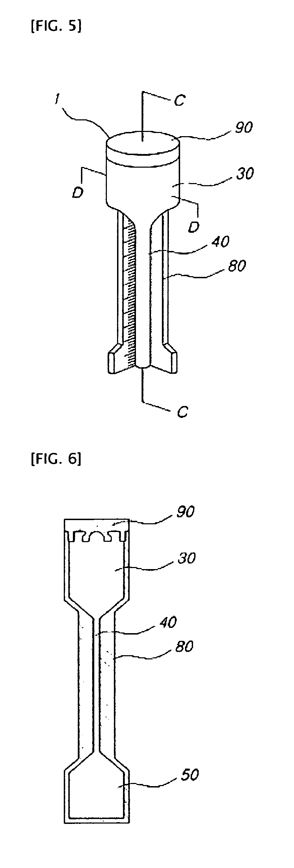

Fig. 5 shows another example of the centrifuge tube of the present invention, wherein the lower chamber in the Fig. 4 is omitted, and the intermediate chamber (40) extends to the bottom of the centrifuge tube, and the centrifuge tube can selectively used depending on the properties of biological solution. That is, the centrifuge tube is configured so as to be suitable for centrifugation of the small volume of the solution with similar whole specific gravity. It may be used when a little finer centrifugation is needed; for example, a little finer and easier separation can be allowed if the centrifuge tube is used in the case that the erythrocyte, blood platelet and leukocyte in the primarily separated blood are similar to each other in compositional ratio.

In the case of the centrifuge tube of such configurations, due to the support pieces (80) formed on the side surface of the intermediate chamber (40) extending to the bottom, the intermediate chamber (40) is prevented from being warped or shaken by pressure of high acceleration of gravity (g) applied to the intermediate chamber (40) at the time of centrifugation, whereby the components of the received blood are arranged in order according to the difference in specific gravity of the respective component, hence the support pieces help to allow a little finer centrifugation. In addition, when the diameter of the intermediate chamber (40) is very small, the chamber is difficult to indicate the volume of the solution, however, the support pieces (80) affords to indicate the volume with the scale. Furthermore, blades are formed at distal end portion of the support pieces (80), which blades extend outwardly long such that the distal end portion can be brought into close contact with the bucket of the centrifugal separator and allow the centrifuge tube removed from the centrifugal separator to be erected on a plane surface, thereby allowing convenient separating operation.

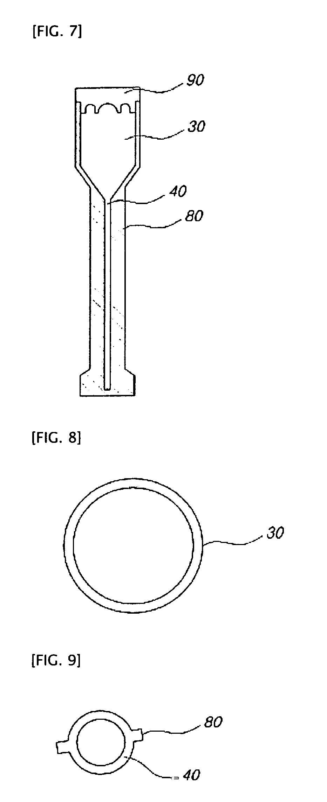

Fig. 6 shows a sectional view taken along line A - A in Fig. 4 and Fig. 7 shows a sectional view taken along line C - C in Fig. 5, wherein the upper chamber (30) and lower chamber (50) have a pentagonal shape in cross section, however, the pentagonal shape is necessarily needed, thus the cross section may be inverted triangular or triangular or in rectangle-like shape, if necessary. Meanwhile, the thickness of bottom surface of the lower chamber (50) is smaller than the thickness of the wall surface so that the bottom surface is slightly flexible, therefore, when the buffy coat layer is caught in the lower chamber (50), pushing of the flexible portion of the bottom surface of the lower chamber (50) causes the buffy coat layer to be moved toward the intermediate layer (40), whereby the separation can be facilitated. Also, as shown in Figs. 5 and 7, lower portions of the support pieces (80) formed on the side surface of the intermediate chamber (40) are formed in such a manner that its ends are extended long and thus can be brought into close contact with the inside of the bucket of centrifugal separator, therefore, the support pieces serve to prevent the centrifuge tube from being shaken at the time of centrifugation.

Figs. 8 and 9 are sectional views taken along line B - B and line D - D, respectively. At least one support pieces (80) are formed on the surface of the intermediate chamber (40) with a small diameter.

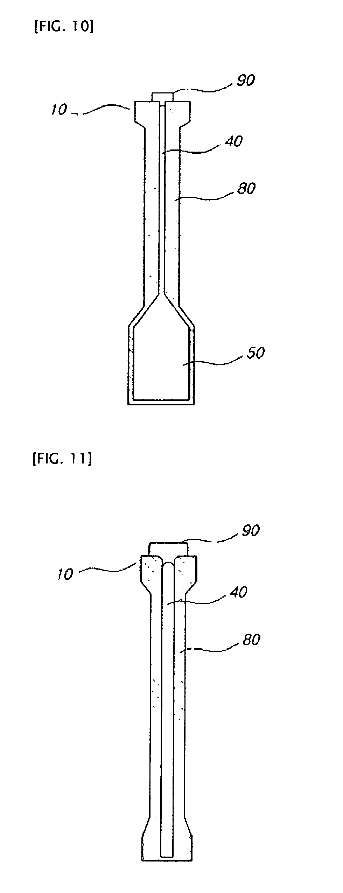

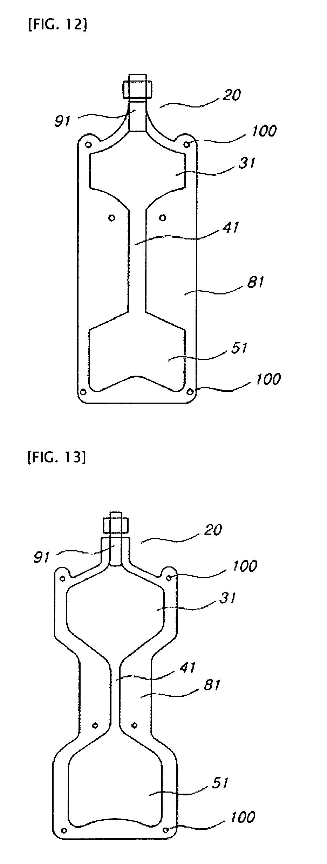

Fig. 10 shows the intermediate chamber (40) whose diameter becomes narrower upward such that when components to be a little more finely separated are distributed above the middle the components can be easily separated. The centrifuge tube shown in Fig. 13 may be conveniently used to detect substance to be separated from the solution of wholly small quantity. In each case described above, the support pieces (80) prevent not only deformation of the centrifuge tube, but also disorder of boundary layer of each centrifuged component due to shaking by close contact of the extended blade portions of the support pieces (80) with the centrifugal bucket.

Figs. 12, 13 and 14 are views showing examples of a blood sampling bag. The blood sampling bag (20) is used for sampling the blood and storing it, and PVC is much used as material for the bag. The blood extracting bag (20) is divided into a upper chamber (31), intermediate chamber (41) and lower chamber (51), and when subjected to centrifugation, the components of the blood are separated from each other according to their specific gravity. At this time, support pieces (81) formed on the intermediate section of the blood sampling bag (20) prevent the elongate intermediate chamber (41) with a small diameter from being torn and deformed due to the high acceleration of gravity (g) generated at the time of centrifugation, and also serve to secure the shape of the intermediate chamber (41) in operation of separating and extracting the each component, thereby preventing the buffy coat layer from being undulated or disordered.

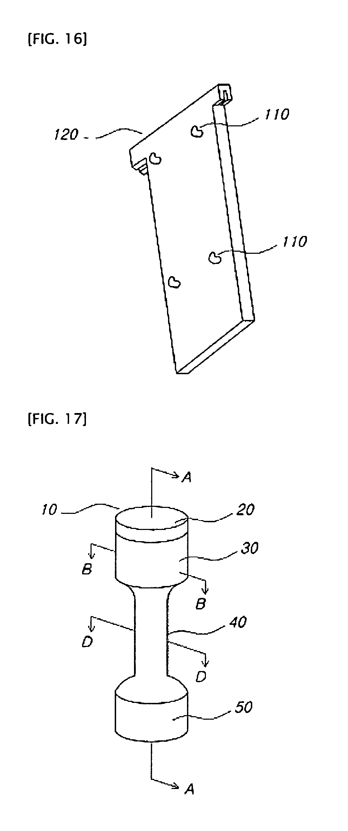

Fig. 16 is a perspective view of a flat bucket (120) caught on a rotor of the centrifugal separator, wherein the blood sampling bag (20) is hung on rings (110) formed on the flat bucket (120). At this time, hanging holes (100) formed in the blood extracting bag (20) correspond to the rings (110) of the flat bucket (120).

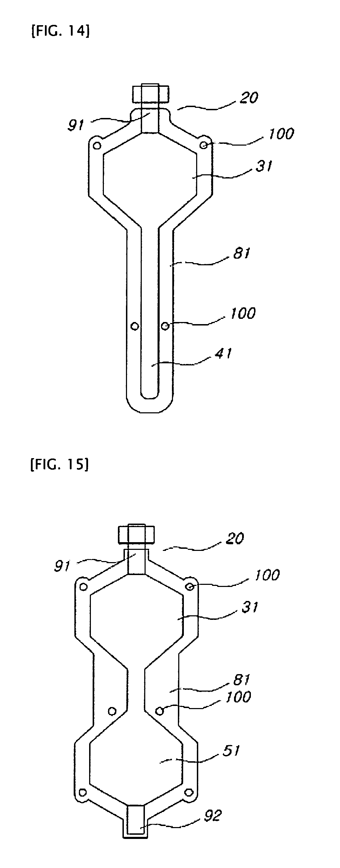

Meanwhile, when the buffy coat layer including the blood platelet layer or leukocyte layer in the centrifuged blood is formed in the lower chamber (51), if the bottom of the lower chamber (51) is slightly pushed, the buffy coat layer is slightly moves to be positioned in the intermediate chamber (41), whereby it can be easily separated by means of a needle or capillary tube. Furthermore, since the blood plasma layer, erythrocyte layer and blood platelet layer are separated from each other in the intermediate chamber (41) with a narrow width or small diameter, interfaces of the layers can be separated by pinching the interface by an elongate forceps, pressing machine, welding machine etc., and if the interface is fused by means of the welding machine etc., the centrifuged components of the blood can be stored without moving it into their separate containers etc, and as shown in Fig. 15, the erythrocyte can be separately extracted through a bottom inlet (92).

Previously added into the centrifuge tube may be water-soluble polymeric compounds precipitating a specific substance, or separation material such as ficoll-hypaque etc., anticoagulant such as heparin, ACDA, CPDA, EDTA etc. or fibrin activation agent such as thrombin, Calcium divalent ion, glass beads, or targeted fluorescent material-separating reagent utilizing antigen-antibody reaction or magnetic microbeads etc. The above-mentioned reference numerals are those indicated in Figs. 1~16.

Fig. 17 shows another example of the centrifuge tube of the present invention wherein the blood is extracted to be centrifuged, the erythrocyte layer with large specific gravity is collected in the lower chamber (50), buffy coat layer of the leukocyte and blood platelet etc. is separated in the intermediate chamber (40). In order to prevent the intermediate chamber (40) from being deformed due to the high acceleration of gravity (g) generated at the time of centrifugation, the thickness of the intermediate chamber (40) with a small diameter is made larger than that of another upper chamber (30) or lower chamber (50) with a large diameter, thereby enduring the high acceleration of gravity (g). Furthermore, inlet portion of the upper chamber (30) may be formed so thick that a stopper (20) can be fitted to the inlet portion. The stopper (20) is provided on the top of the centrifuge tube, which stopper is made of chemical polymeric material such as rubber, silicone, polyethylene, polypropylene or PVC etc. and may be pierced by a needle. The stopper (20) may be coupled to the centrifuge tube in a fitting manner or by forming threads on the tube and also forming threads symmetrical to the threads of the tube on the stopper and screwing the cap into the tube. That is, when centrifuging the biological liquid such as the blood etc., since the liquid such as blood etc. can be charged into the centrifuge tube even without opening of the stopper (20), the centrifugation can be carried out free from inclusion of virus or germs. Therefore, closed operation can be performed in medical equipment or biological instrument requiring strict sterilization, whereby contamination of the specimen, culture or centrifuged substance can be prevented.

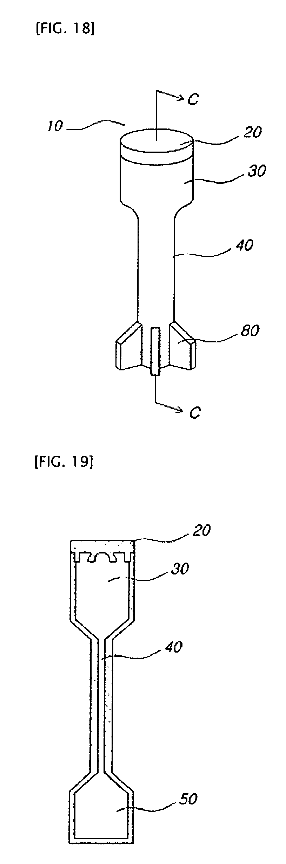

Fig. 18 shows yet another example of the centrifuge tube of the present invention, wherein the lower chamber is omitted and the intermediate chamber (40) extends to the bottom of the centrifuge tube. This centrifuge tube can selectively used depending on the properties of biological solution, and is suitable for centrifugation of the small volume of the solution with similar whole specific gravity. It may be used for a little finer centrifugation; for example, a little finer and easier separation can be allowed if the centrifuge tube is used in the case that the erythrocyte, blood platelet and leukocyte in the primarily separated blood are similar to each other in compositional ratio. By forming the support pieces (80) formed on the lower portion so as to extend outwardly long such that the support pieces can be brought into close contact with the bucket of the centrifugal separator, vibration can be prevented in centrifugation by means of close contact of the support pieces with the bucket, and such support pieces enable the centrifuge tube removed from the centrifugal separator to be erected on a plane surface, thereby assisting in separating operation of each component.

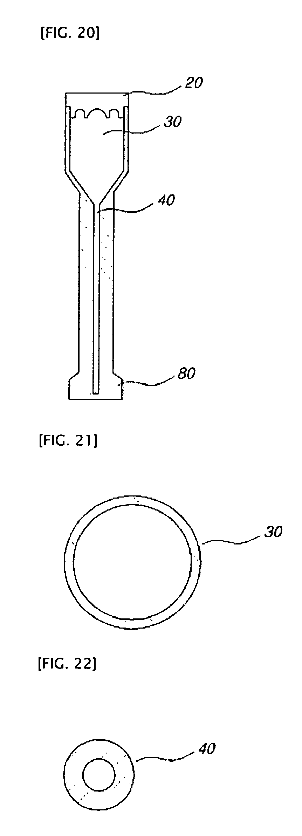

Figs. 19 and 20 are sectional views taken along line A - A and line C - C, respectively, wherein the cross section of the upper chamber (30) and lower chamber (50) are not necessarily in pentagonal shape, thus the cross section may be inverted triangular or triangular or in rectangle-like shape, if necessary. Meanwhile, for the thickness of the lower chamber (50), the lower chamber is made thin to be slightly flexible, therefore, when the buffy coat layer is caught in the lower chamber (50), pushing of the lower chamber (50) causes the buffy coat layer to be moved toward the intermediate layer (40), whereby the separation can be facilitated. Also, as shown in Figs. 18 and 20, the support pieces (80) formed on the lower side of the intermediate chamber (40) are formed so as to be brought into close contact with the inside of the bucket of centrifugal separator, therefore, the support pieces serve to prevent the centrifuge tube from being shaken at the time of centrifugation.

Figs. 21 and 22 are sectional views taken along line B - B and line D - D, respectively. The thickness of the intermediate chamber (40) is larger than that of the upper chamber (30), thus deformation is prevented at the time of centrifugation in which the high acceleration of gravity(g) is applied, and when the buffy coat layer including the blood platelet layer or leukocyte layer in the centrifuged blood is formed in the lower chamber (50), if the bottom of the lower chamber (50) is slightly pushed, the buffy coat layer is slightly moves to be positioned in the intermediate chamber (40), whereby the buffy coat layer it can be easily separated by means of a needle or capillary tube. The above-mentioned reference numerals are those indicated in Figs. 17~22.

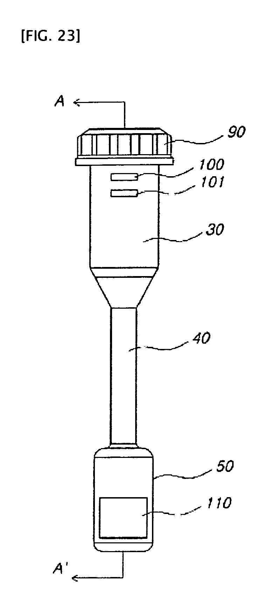

Fig. 23 is a perspective view showing an example of the centrifuge tube of the present invention, wherein when the blood etc. was extracted to be centrifuged, the erythrocyte layer with large specific gravity is collected in the lower chamber (50), the buffy coat layer including stem cells of the leukocyte and blood platelet etc. is collected in the intermediate chamber (40) and blood plasma such as serum is collected in the upper chamber (30).

The upper chamber (30) is provided at certain level on its side surface with indication part such as indication lines (100, 101), indication characters, indication symbols etc. for indicating the volume of the blood charged into the centrifuged tube. In this connection, the indication line (100) indicates the volume of charged peripheral blood for woman, and the indication line (101) indicates the volume of charged peripheral blood for man. By differently charging the peripheral blood for man and woman as described above, since the compositional ratios of the erythrocyte collected in the lower chamber (50) are similar, the centrifuge tube can be used in common both for man and woman without manufacturing separate centrifuge tubes for man and woman. That is, the lower chamber (50) of the centrifuge tube in which the erythrocyte is collected can be formed with a constant volume according to the difference of components of the blood between man and woman. In stead of providing only one indication line (100) and indication line (101), volume indication lines(indication scale) may be provided as metric indication line; as shown on the intermediate chamber in Figs. 4 and 5. In such a case, the volume marking lines can be more conveniently used in that the volume of the respective component can be measured to be seen. The volume marking line is shown to be provided on the intermediate chamber, however, it may be provided on the upper chamber and/or lower chamber. The volume marking line may be also used as the indication line by providing marks for man and woman. Conventionally, as shown in Fig. 3, the volume of the lower chamber (50) was adjusted by providing a separate plug (60) to the lower chamber (50) and tightening and releasing it, which results in increase of production cost, and in some cases for the purpose of saving of cost, the plug (60) is separated and then re-used after washed and sterilized, however, its re-use causes an insanitary problem because it is re-used without complete removal of the blood of other people remaining on a narrow screw. However, employment of the example of the present invention can solve such a problem.

The indication lines (100, 101) indicating the volume of the charged peripheral blood are provided on the surface of the upper chamber (30) in consideration of the facts that ratios of the erythrocyte for man and woman are 47% and 42%, respectively, and the amount of anticoagulant introduced during the process of extracting the peripheral blood is usually slight for heparin, but the anticoagulant such as ACDA is added in ratio of about 10 ~ 15% of the blood. Furthermore, the indication lines (100, 101) are marked as a line having a certain thickness such that the blood can be differently charged depending on man or woman or the amount of the added anticoagulant. Since such indication lines (100, 101) are marked with a certain thickness, a side above the line indicates the case that the small amount of anticoagulant is introduced and a side below the line indicates the case that the much amount of anticoagulant is introduced, although the line (100) or line (101) is the one same line. Furthermore, though not shown in the drawings, by marking the indication lines with color, symbols or characters, the buffy coat layer including desired stem cells can be positioned in any one portion of the intermediate chamber (40) at the time of separating operation, whereby efficient separating operation of the stem cells can be performed. Such indication lines (100, 100) may be marked separately from the scale indicating the total volume or together with the scale indicating the total volume. It is a matter of course that the indication lines (100, 101) may be differently marked if the substance charged into the centrifuge tube is not the peripheral blood of the human being, but other substance to be separated.

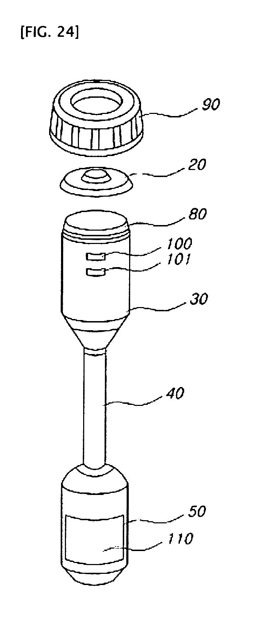

Furthermore, as shown in Fig. 24, a stopper (20) is provided on the top of the centrifuge tube, which stopper is made of chemical polymeric material such as rubber, silicone, polyethylene, polypropylene or PVC etc. and may be pierced by a needle. The stopper (20) may be coupled to the centrifuge tube in a fitting manner or by forming threads on the centrifuge tube and also forming threads symmetrical to the threads of the tube on the stopper and screwing the stopper into the tube. The stopper (20) is formed in such a way that its top portion is slightly raised, so the stopper can be well wiped with alcohol cotton prior to insertion of a needle, without a need to open the cap (90). The inside of the stopper is contrarily concave, so that specific substance in the centrifuge tube can be collected in the concave portion.

Furthermore, the cap (90) with open top is provided separately from the stopper (20), therefore, the stopper (20) can be prevented from being opened when the needle inserted into the stopper (20) is withdrawn again. The cap (90) is formed on its inner surface with a settlement groove (81), which engages and couples with a corresponding settlement shoulder (80) of the centrifuge tube. By such a configuration, the peripheral blood can be charged into the centrifuge tube and desired component substance can be separated and extracted without opening the stopper (20) or cap (90), whereby an effect is provided that inclusion of virus or germ is prevented. Therefore, closed operation can be performed in medical equipment or biological instrument requiring strict sterilization, whereby contamination of the specimen, culture or centrifuged substance can be prevented. Here, it is a matter of course that the settlement shoulder (80) and settlement groove (81) are formed with threads etc. Furthermore, for the thickness of the lower chamber (50), the lower chamber is made thinner than other two chambers to be slightly flexible, therefore, when the buffy coat layer is caught in the lower chamber (50), pushing of the flexible portion of the bottom surface of the lower chamber (50) causes the buffy coat layer to be moved toward the intermediate layer (40), whereby the separation can be facilitated. Though not shown in drawings, support pieces may be formed on the surface of the intermediate chamber (40) with a small diameter.

For a method of extracting the buffy coat layer by inserting a syringe needle, an end of the needle is inserted upon insertion of the syringe so as to reach the buffy coat layer and then sucks it. The last portion of the buffy coat layer is bounded by the erythrocyte under the layer with a thin layer therebetween, and at this time if an end of the needle is rotated describing a circle inside the wall surface of the intermediate chamber, due to centrifugal force, the heavy erythrocyte adhere to the wall surface of the intermediate chamber while relatively light buffy coat layer is collected in the central portion, and at this time if a method is used of sucking the centrally collected buffy coat layer with the end of the needle, the remaining minute amount of the buffy coat layer can be extracted.

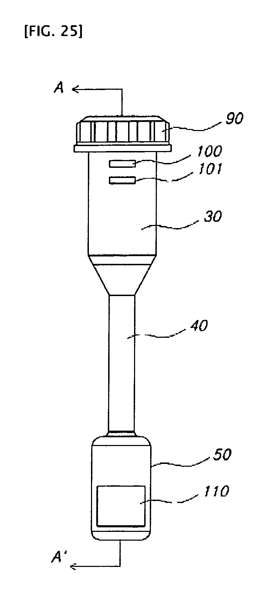

As shown in Fig. 25, the surface of the centrifuge tube is formed with display surface (110) on which name or test date etc. of the tested person may be recorded. Due to such a display surface (110), the testing person can be prevented from making a wrong diagnosis by confusing the tested persons when many tests are concurrently carried out.

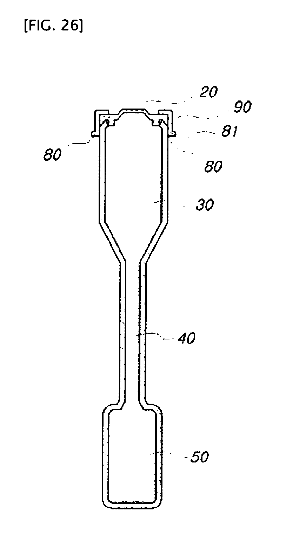

Fig. 26 is a sectional view taken along line A - A' in Fig. 25, wherein the settlement shoulder (80) is formed on the surface of the centrifuge tube and a settlement groove (81) is formed at corresponding location on the inner surface of the cap (90), which groove and shoulder engage and couple with each other. The above-mentioned reference numerals are those indicated in Figs. 23 ~ 26.

According to the present invention, previously added into the centrifuge tube may be water-soluble polymeric compounds precipitating a specific substance, or separation material such as ficoll-hypaque etc., anticoagulant such as heparin, ACDA, CPDA, EDTA etc. or fibrin activation agent such as thrombin, Calcium divalent ion, glass beads, or targeted fluorescent material-separating reagent utilizing antigen-antibody reaction or magnetic microbeads etc, therefore, the centrifuge tube can be conveniently applied for required use.

Though various examples has been described as above, many various examples other than those can be easily represented based on the technical concepts of the present invention, and such examples are also naturally included in the scope of the present invention.

The present invention centrifuges the blood to separate specific component, and utilizes the separated component in medical science and biotechnology. Devices of the present invention assist in economical, safe and efficient separation of biological substance shielded from contamination, including biological contamination, occurring during a separating process in separation, cultivation and regeneration of cells.

## Claims

1. A centrifuge tube having an outer wall for receiving flowable substance in inner space and for separating components of the flowable substance received in the inner space according to specific gravity, wherein the centrifuge tube comprises an upper chamber having an open top inlet and constant inner volume; an intermediate chamber communicating with the upper chamber at lower end thereof and having a smaller diameter than the upper chamber; an lower chamber communicating with the intermediate chamber at lower end thereof and having a larger diameter than the intermediate chamber; and at least one support pieces formed integrally with the an outer wall of the intermediate chamber.

2. A centrifuge tube having an outer wall for receiving flowable substance in inner space and for separating components of the flowable substance received in the inner space according to specific gravity, wherein the centrifuge tube comprises an upper chamber having an open top inlet and constant inner volume; an intermediate chamber communicating with the upper chamber at lower end thereof and having a smaller diameter than the upper chamber; and at least one support pieces formed integrally with the an outer wall of the intermediate chamber.

3. The centrifuge tube according to claim 1 or 2, wherein the tube further comprises a stopper made of flexible material and closing the inlet of the upper chamber.

4. The centrifuge tube according to claim 3, wherein the stopper is made of one of rubber, silicone, PVC, polyethylene, polypropylene or polymer.

5. The centrifuge tube according to claim 2, wherein lower portions of the support pieces extend to a bucket of a centrifugal separator.

6. The centrifuge tube according to claim 1, wherein the thickness of bottom surface of the lower chamber is smaller than that of side surface thereof, so that the bottom surface is flexible.

7. A centrifugal pack having an outer shell for receiving flowable substance in inner space and for separating components of the flowable substance received in the inner space according to specific gravity, wherein the centrifugal pack comprises an upper chamber having an open top inlet and constant inner volume; an intermediate chamber communicating with the upper chamber at lower end thereof and having a smaller diameter than the upper chamber; and an lower chamber communicating with the intermediate chamber at lower end thereof and having a larger diameter than the intermediate chamber, and wherein hanging holes are formed in outer shell which forms the upper chamber, intermediate chamber and lower chamber, and these hanging holes are hung on a flat bucket having rings.

8. The centrifugal pack according to claim 7, wherein a lower inlet is formed at a lower portion of the centrifugal pack.

9. A centrifuge tube having an outer wall for receiving flowable substance in inner space and for separating components of the flowable substance received in the inner space according to specific gravity, wherein the centrifuge tube comprises an upper chamber having an open top inlet and constant inner volume; an intermediate chamber communicating with the upper chamber at lower end thereof and having a smaller diameter than the upper chamber; and an lower chamber communicating with the intermediate chamber at lower end thereof and having a larger diameter than the intermediate chamber, and wherein an outer wall of the intermediate chamber is thicker than outer walls of the upper and lower chambers.

10. A centrifuge tube having an outer wall for receiving flowable substance in an inner space and for separating components of the flowable substance received in the inner space according to specific gravity, wherein the centrifuge tube comprises an intermediate chamber having an open top inlet and constant inner volume; and an lower chamber communicating with the intermediate chamber at lower end thereof and having a larger diameter than the intermediate chamber, and wherein an outer wall of the intermediate chamber is thicker than an outer wall of the lower chamber.

11. The centrifuge tube according to claim 9 or 10, wherein a stopper of the centrifuge tube is made of rubber, silicone, PVC, polyethylene, polypropylene or polymeric plastic or combinations thereof.

12. The centrifuge tube according to claim 9 or 10, wherein at least one support pieces are formed on side surface of the intermediate chamber, or at least one support pieces are formed on the side surface of the intermediate chamber so as to extend to a bucket of a centrifugal separator.

13. The centrifuge tube according to any one of claims 1 to 12, wherein added into the chambers is fibrin activation agent such as thrombin, Calcium divalent ion, glass beads, or anticoagulant such as heparin, ACDA, CPDA, EDTA etc. or water-soluble polymeric compounds precipitating a specific substance, or separation material such as ficoll-hypaque etc., or targeted fluorescent material-separating reagent utilizing antigen-antibody reaction or magnetic microbeads.

14. A centrifuge tube comprising an intermediate chamber with a small diameter, an lower chamber with relatively large diameter and an upper chamber, wherein indication part for indicating the volume of the object to be centrifuged and partial volume of components thereof is formed on outer surface of the intermediate chamber, upper chamber or lower chamber.

15. A centrifuge tube comprising an intermediate chamber with a small diameter, an lower chamber with relatively large diameter and an upper chamber, wherein a upper end of the upper chamber is closed by a stopper of which central portion is convex.

16. The centrifuge tube according to claim 15, wherein a settlement shoulder is formed on upper end of the upper chamber, and a cap is further provided which has a settlement groove engaging the settlement shoulder, thereby preventing easy separation of the stopper from the upper chamber.

17. The centrifuge tube according to claim 15, wherein the stopper of the upper chamber is made of rubber or silicone and is formed in such a manner that outer central portion of the stopper is convex and inner portion thereof is concave.

18. The centrifuge tube according to claim 14 or 15, wherein the thickness of the outer wall forming the lower chamber is smaller than those of outer walls of the upper and intermediate chambers, so that the outer wall of the lower chamber is flexible.

19. The centrifuge tube according to any one of claims 14 to 18, wherein previously added into the centrifuge tube is a predetermined amount of anticoagulant such as heparin, ACDA, CPDA, EDTA etc. or water-soluble polymeric compounds precipitating a specific substance, or separation material such as ficoll-hypaque etc., or fibrin activation agent such as thrombin, Calcium divalent ion, glass beads, or targeted fluorescent material-separating reagent utilizing antigen-antibody reaction or magnetic microbeads.

20. A centrifuge tube having an upper chamber, intermediate chamber and lower chamber, wherein the volume of the lower chamber accounts for about 36% of the total volume of the centrifuge tube, and the volume of the intermediate chamber accounts for about 10 ± 2% of the total volume, thus the component to be extracted is collected in the intermediate chamber.

21. The centrifuge tube according to claim 20, wherein in the case of the centrifuge tube applied to a centrifugal adaptor for 15 ml type, the outer diameter of the upper chamber is 16 ± 2 mm, and the outer diameter of the lower chamber is 16 ± 2 mm, and the outer diameter of the intermediate chamber is 6 ± 2 mm.

22. The centrifuge tube according to claim 20, wherein in the case of the centrifuge tube applied to centrifugal adaptor for 50 ml type, the outer diameter of the upper chamber is 28 ± 2 mm, the outer diameter of the lower chamber is 28 ± 2 mm, and the outer diameter of the intermediate chamber is 6 ± 2 mm.
